# EUROPEAN PATENT APPLICATION

(11) **EP 4 721 682 A2**
(43) Date of publication of application: **08.04.2026**
(21) Application number: 25206268.2
(22) Date of filing: 02.10.2025
(51) Int. Cl.: A61B 17/32

(54) **SURGICAL DEVICE**

(30) Priority: 04.10.2024 TW 113137853
(71) Applicant: Advanced Medical Design Co., Ltd., New Taipei City 248, R.O.C. (TW)
(72) Inventor: YEH, Yen-Ming, New Taipei City, R.O.C. (TW); YU, Ping-Sheng, New Taipei City, R.O.C. (TW)
(74) Representative: dompatent

(57) **Abstract**

A surgical device (10) including a housing (100), a circuit assembly (200), a driving component (300), a tool (400) and an input assembly (500). The circuit assembly is disposed in the housing. The driving component is disposed in the housing and electrically connected to the circuit assembly. The tool is connected to the driving component and configured to be driven by the driving component. The tool sticks out of a first end part (110) of the housing. The input assembly includes a keypad (510) and at least one button switch (520). The at least one button switch is disposed on the keypad and electrically connected to the circuit assembly. The keypad is disposed on the housing and exposed to the outside.

## Description

### Technical Field

The disclosure relates to a surgical device, more particularly to a surgical device including at least one tool.

### Background

In general, in order to control the tool of the surgical device, mechanical push buttons are disposed on the surgical device to facilitate the adjustment of the surgical device. In addition, during the surgery performed by using the surgical device, a liquid, such as water, is required to be sprayed onto the affected part. Thus, additional waterproof structures are usually required to be disposed around the mechanical push buttons to effectively prevent such liquid from penetrating into the surgical device around the mechanical push buttons.

However, recently, for hygiene reasons, the surgical device is gradually designed to be disposable. For such disposable surgical device, if the aforementioned mechanical push buttons and waterproof structures are adopted, the surgical device will be heavy and costly. In this way, the surgical device is not allowed to be lightweight and cheap while effectively preventing the liquid from penetrating into the surgical device.

### SUMMARY

The disclosure provides a surgical device including at least one button switch disposed on a keypad, thereby allowing the surgical device to be lightweight and cheap while effectively preventing a liquid from penetrating into the surgical device.

One embodiment of this disclosure provides a surgical device including a housing, a circuit assembly, a driving component, a tool and an input assembly. The circuit assembly is disposed in the housing. The driving component is disposed in the housing and electrically connected to the circuit assembly. The tool is connected to the driving component and configured to be driven by the driving component. The tool sticks out of a first end part of the housing. The input assembly includes a keypad and at least one button switch. The at least one button switch is disposed on the keypad and electrically connected to the circuit assembly. The keypad is disposed on the housing and exposed to the outside.

According to the surgical device disclosed by above embodiments, the button switch is disposed on the keypad. Thus, the liquid is effectively prevented from penetrating into the surgical device by the excellent sealing between the keypad and the housing, thereby allowing additional waterproof structures around the button switch to be omitted. In this way, the surgical device is allowed to be lightweight and cheap while effectively preventing the liquid from penetrating into the surgical device.

### BRIEF DESCRIPTION OF THE DRAWINGS

The present disclosure will become better understood from the detailed description given herein below and the accompanying drawings which are given by way of illustration only and thus are not intending to limit the present disclosure and wherein:
FIG. 1 is a schematic perspective view of a surgical device according to a first embodiment of the disclosure and a power source;
FIG. 2 is a cross-sectional view of the surgical device in FIG. 1;
FIG. 3 is a partially exploded view of the surgical device in FIG. 1;
FIG. 4 is a partially enlarged side view of the surgical device in FIG. 1; and
FIG. 5 is a partially enlarged side view of a surgical device according to a second embodiment of the disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

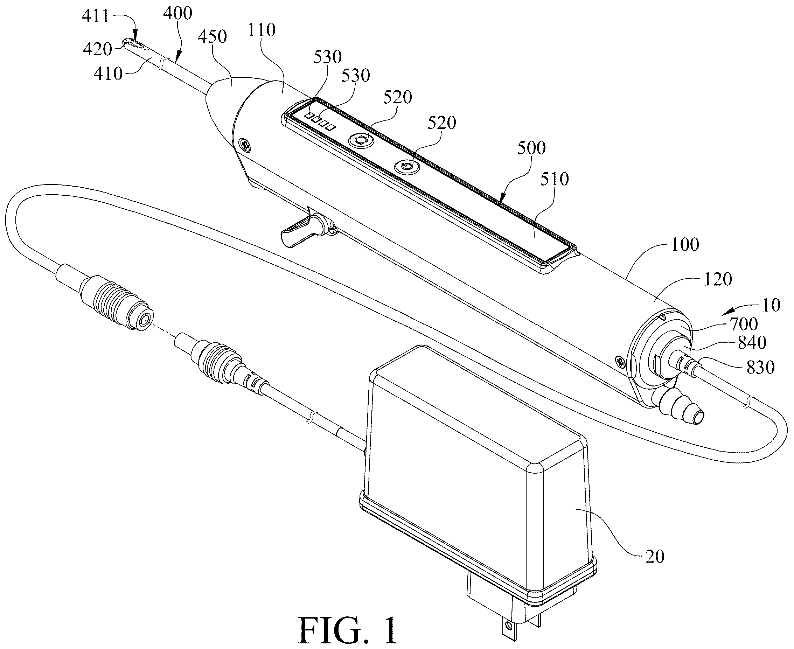

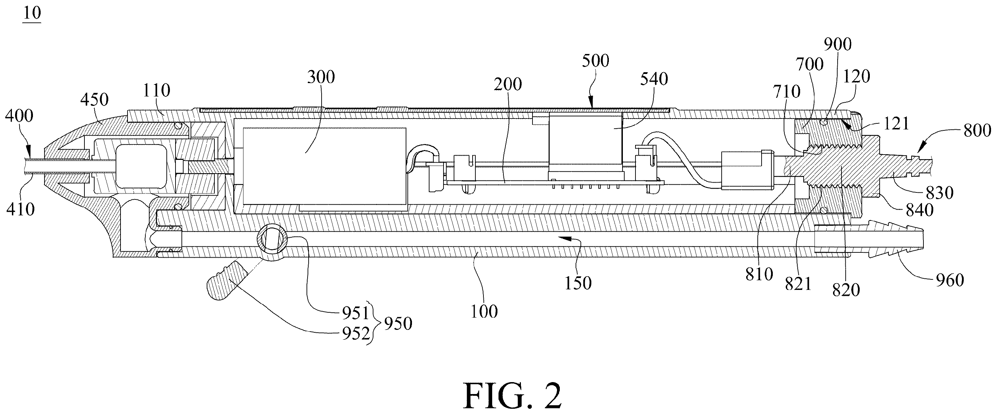

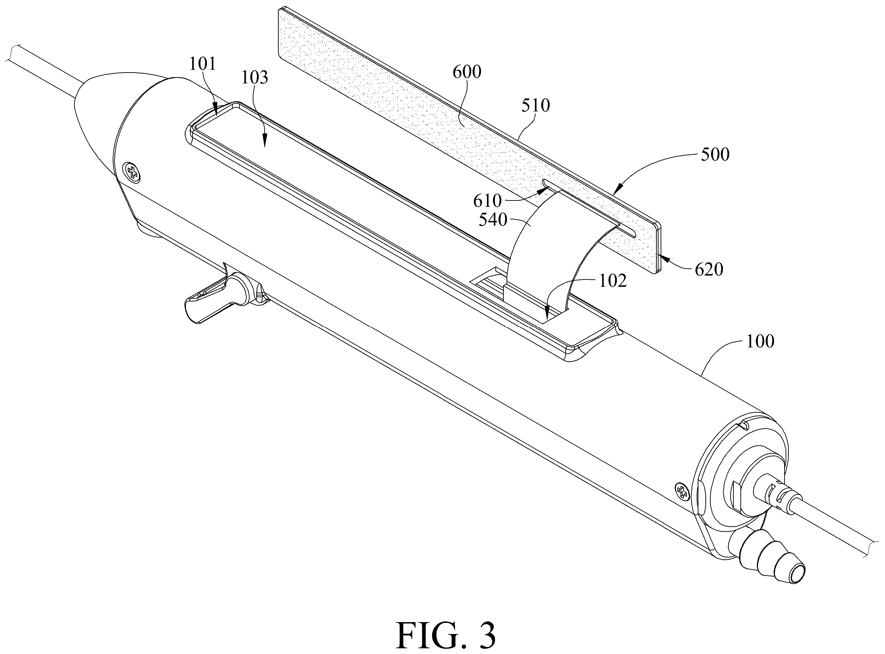

Please refer to FIGS. 1 to 3. FIG. 1 is a schematic perspective view of a surgical device 10 according to a first embodiment of the disclosure and a power source 20. FIG. 2 is a cross-sectional view of the surgical device 10 in FIG. 1. FIG. 3 is a partially exploded view of the surgical device 10 in FIG. 1.

In this embodiment, the surgical device 10 includes, for example, a housing 100, a circuit assembly 200, a driving component 300, a tool 400, an input assembly 500, an adhesive layer 600, an assembling barrel 700, a cable 800 and a sealing ring 900. The surgical device 10 may, for example, cooperate with an endoscope to perform surgery on an affected part (not shown).

The housing 100 has a first end part 110 and a second end part 120 opposite to each other. The second end part 120 has a mounting opening 121.

The circuit assembly 200 is disposed in the housing 100. The driving component 300 is, for example, a motor. The driving component 300 is disposed in the housing 100 and electrically connected to the circuit assembly 200.

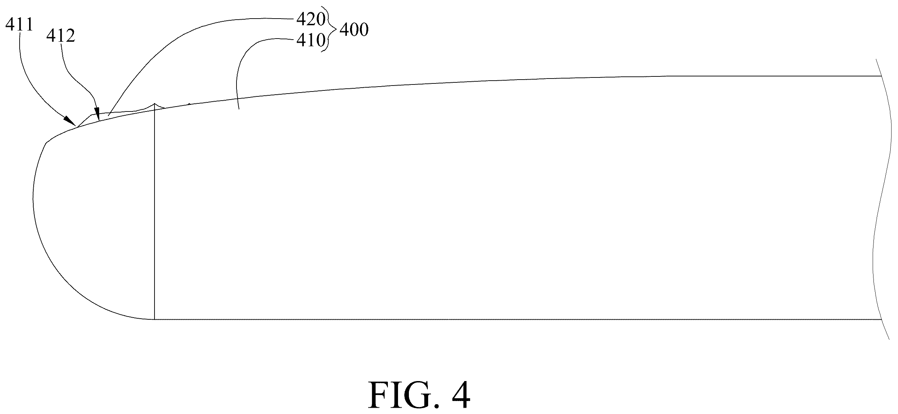

Please refer to FIGS. 2 and 4. FIG. 4 is a partially enlarged side view of the surgical device 10 in FIG. 1. The tool 400 is connected to the driving component 300 and is configured to be driven by the driving component 300. The tool 400 sticks out of the first end part 110 of the housing 100. In detail, the tool 400 includes, for example, an outer tube 410 and a knife 420. The outer tube 410 sticks out of the first end part 110. An end of the outer tube 410 located away from the housing 100 has a notch 411. The knife 420 is disposed in the outer tube 410 and exposed to the outside from the notch 411. For example, the knife 420 may have a serrated shape and be configured to shred the affected part. The knife 420 is connected to the driving component 300 and is configured to be driven by the driving component 300. In addition, in this embodiment, for example, a resting surface 412 of the outer tube 410 forming the notch 411 is a convex surface to rest on the affected part more tightly. Moreover, in this embodiment, the surgical device 10 may, for example, further include a front cover 450. The front cover 450 is, for example, removably disposed on the first end part 110. The outer tube 410 is disposed on the front cover 450. In other embodiments, the front cover and the housing may be integrally formed as a single piece.

Please refer to FIGS. 1 to 3. The input assembly 500 includes a keypad 510, a plurality of button switches 520, a plurality of light emitting components 530 and a cable 540. The button switches 520 and the light emitting components 530 are disposed on the keypad 510, and are electrically connected to the circuit assembly 200 in the housing 100 via, for example, a circuit assembly (not shown) in the keypad 510 and the cable 540. The keypad 510 is disposed on the housing 100 and exposed to the outside. In detail, in this embodiment, the housing 100 has, for example, a recess 101 and a through hole 102. The through hole 102 is located on a bottom surface 103 forming the recess 101. The keypad 510 is fixed in the recess 101. The adhesive layer 600 is located on a side of the keypad 510 located close to the bottom surface 103 and has an opening 610. The opening 610 is spaced apart from an edge 620 of the adhesive layer 600. The cable 540 is disposed through the opening 610 and the through hole 102.

Specifically, one of the button switches 520 is configured to start or stop the drive of the driving component 300 on the knife 420, or to change the direction along which the driving component 300 drives the knife 420. Another button switch 520 is configured to adjust the rotational speed of the driving component 300 and thus the rotational speed of the knife 420. The light emitting components 530 indicate the value of the current rotational speed by cooperating with number markings on the keypad 510 adjacent thereto. Note that in other embodiments, the input assembly may include one button switch, and the input assembly may not include the light emitting component.

By fixing the keypad 510 in the recess 101, the sealing between the keypad 510 and the housing 100 is improved.

Further, since the opening 610 of the adhesive layer 600 configured for the cable 540 to be disposed therethrough is spaced apart from the edge 620, there is no gap, slit, hole or clearance in the adhesive layer 600, thereby preventing a liquid (e.g., water) from flowing from the outside of the housing 100 into the opening 610.

The disclosure is not limited by the manner for the keypad 510 to be fixed on the housing 100. In other embodiments, the keypad may be fixed to the housing via magnet, welding, soldering, latch, lock or any other suitable manners.

The keypad 510 may, for example, be in a sheet shape and have a rectangular cross section, but the disclosure is not limited thereto. In other embodiments, the keypad may be in a sheet shape and have a circular, square or irregular cross section.

The button switches 520 and the light emitting components 530 may, for example, be in a circular, square, triangular, oval, or an arbitrary shape.

Please refer to FIGS. 1 and 2. The assembling barrel 700 is disposed in the mounting opening 121 and disposed through the second end part 120. The assembling barrel 700 has, for example, an inner screw thread 710. The cable 800 includes, for example, a first cable part 810, an assembling part 820, a second cable part 830 and a pad part 840. The assembling part 820 connects the first cable part 810 and the second cable part 830, and is located between the first cable part 810 and the second cable part 830. The pad part 840 connects the assembling part 820 and the second cable part 830, and is located between the assembling part 820 and the second cable part 830. The assembling part 820 has an outer screw thread 821. The assembling part 820 is disposed through the assembling barrel 700, and the inner screw thread 710 is screwed into the outer screw thread 821. In addition, in this embodiment, the hardness of the assembling part 820 is, for example, smaller than the hardness of the assembling barrel 700, thereby further improving the sealing between the assembling part 820 and the assembling barrel 700 that are assembled. The pad part 840 rests on a side of the assembling barrel 700 located away from the first end part 110. The first cable part 810 is electrically connected to the circuit assembly 200. The second cable part 830 is electrically connected to the external power source 20, such as a transformer. The overall length of the cable 800 is about, for example, 3 meters.

With the assembling barrel 700 and the cable 800 assembled to each other, the cable 800 functions as an extension cord of the external power source 20. Thus, although the surgical device 10 is disposable, the external power source 20 is still allowed to be reusable. In this way, the external power source 20, such as a transformer, is not required to be thrown away after the surgery is completed, and thus the cost for performing the surgery by using the surgical device 10 is reduced.

The sealing ring 900 is clamped between the assembling barrel 700 and the second end part 120 of the housing 100 so as to improve the sealing between the assembling barrel 700 and the second end part 120 of the housing 100.

In addition, please refer to FIG. 2. The surgical device 10 may, for example, further include a valve assembly 950 and a joint 960. The housing 100 has, for example, a channel 150 connected to the outer tube 410. The valve assembly 950 includes a valve part 951 and a handheld part 952 connected to each other. The valve part 951 is disposed in the channel 150. The handheld part 952 is configured to be moved to open or close the valve part 951. The joint 960 is disposed on an end of the channel 150, and is, for example, connected to a suction device (not shown) via a tube (not shown). In this way, when the handheld part 952 is moved to open the valve part 951, the suction device sucks the objection in the outer tube 410 via the channel 150, the joint 960 and the tube.

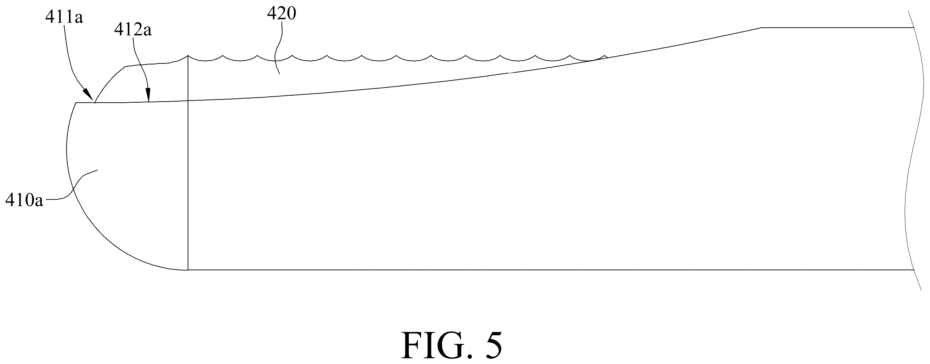

Note that the disclosure is not limited by the curved shape of the resting surface of the outer tube forming the notch. Please refer to FIG. 5. FIG. 5 is a partially enlarged side view of a surgical device according to a second embodiment of the disclosure. In this embodiment, a resting surface 412a of an outer tube 410a forming a notch 411a is, for example, a concave surface. The outer tube 410a may replace the outer tube 410 of the first embodiment to be included in the surgical device 10 of the first embodiment.

According to the surgical device disclosed by above embodiments, the button switch is disposed on the keypad. Thus, the liquid is effectively prevented from penetrating into the surgical device by the excellent sealing between the keypad and the housing, thereby allowing additional waterproof structures around the button switch to be omitted. In this way, the surgical device is allowed to be lightweight and cheap while effectively preventing the liquid from penetrating into the surgical device.

It will be apparent to those skilled in the art that various modifications and variations can be made to the present disclosure. It is intended that the specification and examples be considered as exemplary embodiments only, with a scope of the disclosure being indicated by the following claims and their equivalents.

## Claims

1. A surgical device (10), comprising:
a housing (100);
a circuit assembly (200), disposed in the housing;
a driving component (300), disposed in the housing and electrically connected to the circuit assembly;
a tool (400), connected to the driving component and configured to be driven by the driving component, wherein the tool sticks out of a first end part (110) of the housing; and
an input assembly (500), comprising a keypad (510) and at least one button switch (520), wherein the at least one button switch is disposed on the keypad and electrically connected to the circuit assembly, and the keypad is disposed on the housing and exposed to the outside.

2. The surgical device according to claim 1, wherein the housing has a recess (101), and the keypad is fixed in the recess.

3. The surgical device according to claim 2, further comprising an adhesive layer (600), wherein the housing further has a through hole (102) located on a bottom surface (103) forming the recess, the adhesive layer is located on a side of the keypad located close to the bottom surface and has an opening (610), the opening is spaced apart from an edge (620) of the adhesive layer, the input assembly further comprises a cable (540), the cable is disposed through the opening and the through hole, and electrically connects the at least one button switch and the circuit assembly.

4. The surgical device according to claim 1, further comprising an assembling barrel (700) and a cable (800), wherein the housing further has a second end part (120) opposite to the first end part, the tool sticks out of the first end part, the second end part has a mounting opening (121), the assembling barrel is disposed in the mounting opening and disposed through the second end part, the assembling barrel has an inner screw thread (710), the cable comprises a first cable part (810), an assembling part (820) and a second cable part (830), the assembling part connects the first cable part and the second cable part, and is located between the first cable part and the second cable part, the assembling part has an outer screw thread (821), the assembling part is disposed through the assembling barrel, the inner screw thread is screwed into the outer screw thread, the first cable part is electrically connected to the circuit assembly, and the second cable part is electrically connected to an external power source (20).

5. The surgical device according to claim 4, further comprising a sealing ring (900) clamped between the assembling barrel and the second end part of the housing.

6. The surgical device according to claim 4, wherein a hardness of the assembling part is smaller than a hardness of the assembling barrel.

7. The surgical device according to claim 4, wherein the cable further comprises a pad part (840), the pad part connects the assembling part and the second cable part and is located between the assembling part and the second cable part, and the pad part rests on a side of the assembling barrel located away from the first end part.

8. The surgical device according to claim 1, wherein the tool comprises an outer tube (410) and a knife (420), the outer tube sticks out of the first end part of the housing, an end of the outer tube located away from the housing has a notch (411), and the knife is disposed in the outer tube and exposed to the outside from the notch.

9. The surgical device according to claim 8, wherein a resting surface (412, 412a) of the outer tube forming the notch is a curved surface and configured to rest on an affected part.

10. The surgical device according to claim 9, wherein the resting surface (412) of the outer tube forming the notch is a convex surface.
